(19) Europäisches Patentamt
European Patent Office
Office européen des brevets

(11) EP 2 516 400 B1

(12) **FASCICULE DE BREVET EUROPEEN**

(45) Date de publication et mention de la délivrance du brevet:
**22.03.2017 Bulletin 2017/12**

(21) Numéro de dépôt: **10808918.6**

(22) Date de dépôt: **22.12.2010**

(51) Int Cl.:
***C07D 213/74*** (2006.01) ***A61K 31/44*** (2006.01)
***A61K 31/4409*** (2006.01) ***A61P 5/26*** (2006.01)
***A61P 5/28*** (2006.01)

(86) Numéro de dépôt international:
**PCT/FR2010/052874**

(87) Numéro de publication internationale:
**WO 2011/077046 (30.06.2011 Gazette 2011/26)**

# (54) DERIVES PHENOLIQUES, ET LEUR UTILISATION PHARMACEUTIQUE OU COSMETIQUE

PHENOLDERIVATE UND IHRE PHARMAZEUTISCHE ODER KOSMETISCHE VERWENDUNG

PHENOL DERIVATIVES AND THE PHARMACEUTICAL OR COSMETIC USE THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **23.12.2009 US 282151 P**
**23.12.2009 FR 0959477**

(43) Date de publication de la demande:
**31.10.2012 Bulletin 2012/44**

(73) Titulaire: **Galderma Research & Development**
**06410 Biot (FR)**

(72) Inventeurs:
- **POINSARD, Cédric**
  **F-06130 Le Plan de Grasse (FR)**
- **COLLETTE, Pascal**
  **F-06110 Le Cannet (FR)**
- **MAUVAIS, Pascale**
  **F-06600 Antibes (FR)**
- **LINGET, Jean-Michel**
  **F-67230 Benfeld (FR)**
- **RETHORE, Sandrine**
  **F-06560 Valbonne (FR)**

(74) Mandataire: **Sekhri, Redha et al**
**Cabinet Becker & Associés**
**25, rue Louis le Grand**
**75002 Paris (FR)**

(56) Documents cités:
**WO-A1-2005/042464** **WO-A2-2006/010637**
**DE-U1- 20 217 957** **US-A- 4 578 390**

- **PATHAK V ET AL: "Antiandrogenic phenolic constituents from Dalbergia cochinchinensis", PHYTOCHEMISTRY, PERGAMON PRESS, GB LNKD- DOI:10.1016/S0031-9422(97)00429-9, vol. 46, no. 7, 1 décembre 1997 (1997-12-01), pages 1219-1223, XP004293635, ISSN: 0031-9422**
- **FRANÇOISE PARIS ET AL: "PHENYLPHENOLS, BIPHENOLS, BISPHENOL-A AND 4-TERT-OCTYLPHENOL EXHIBIT ALPHA AND BETA ESTROGEN ACTIVITIES AND ANTIANDROGEN ACTIVITY IN REPORTER CELL LINES", MOLECULAR AND CELLULAR ENDOCRINOLOGY, ELSEVIER IRELAND LTD, IE LNKD- DOI:10.1016/S0303-7207(02)00094-1, vol. 193, no. 1-2, 31 juillet 2002 (2002-07-31), pages 43-49, XP009082517, ISSN: 0303-7207**
- **SCHILLER, CLAUS D. ET AL: "3-(Cyclohexenonyl)-4-(4-hydroxyphenyl)hexanes: antiandrogens derived from the estrogen hexestrol", ARCHIV DER PHARMAZIE, vol. 323, no. 7, 1990, pages 417-420, XP002609206,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 516 400 B1

## Description

La présente description a pour objet des composés de formule générale :

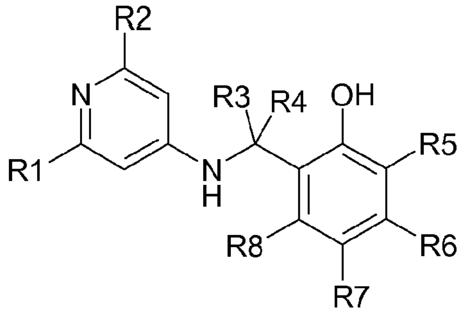

(I)

Et leur utilisation cosmétique ou pharmaceutique.

La présente invention se propose de fournir de nouveaux dérivés phénoliques, qui soient de puissants modulateurs du récepteur aux androgènes.

Parmi les documents de l'art antérieur décrivant des molécules modulant l'activité du récepteur aux androgènes, on peut, par exemple, citer les phenylimidazolines décrites dans la demande de brevet EP580459, ou la demande WO200542464.

Décrits ici sont des dérivés phénoliques qui répondent à la formule générale (I) suivante :

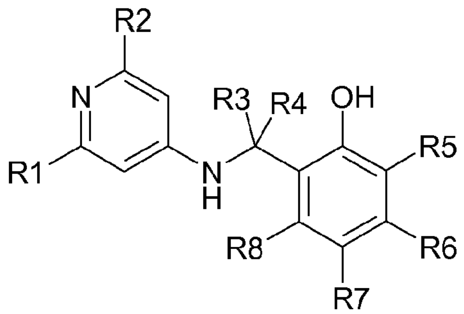

(I)

dans laquelle :

- $R_1$ représente un groupe $C_{1-6}$ alkyle, $C_{3-7}$ cycloalkyle, $C_{1-6}$ alkyloxy, $-S(O)_m-C_{1-6}$-alkyle, $C_{1-6}$ fluoroalkyle, $C_{1-6}$ fluoroalkyloxy, $-(CH_2)_n-C_{3-9}$ cycloalkyle, $-(CH2)_n-C_{3-9}$ cycloalkyle, $C_{2-6}$ alkyle-OH, $-(CH_2)_{n-C1-6}$ alkyloxy, $-(CH_2)_n-C_{1-6}$ fluoroalkyle,$-(CH_2)_p-O-C_{1-6}$ fluoroalkyle, $COR_a$, CN, $NO_2$, $NR_9R_{10}$, un halogène ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_b$ identiques ou différents
- $R_2$ représente un groupe $C_{1-6}$ alkyle, $C_{3-7}$ cycloalkyle, $C_{1-6}$ alkyloxy, $-S(O)_f-C_{1-6}$, alkyle, $C_{1-6}$ fluoroalkyle, $C_{1-6}$ fluoroalkyloxy, $-(CH_2)_l-C_{3-9}$ cycloalkyle, $-(CH_2)_l-C_{3-9}$ cycloalkyle, $C_{2-6}$ alkyle-OH, $-(CH_2)_l-C_1-_6$ alkyloxy, $-(CH_2)_l-C_{1-6}$ fluoroalkyle,$-(CH_2)_q-O-C_1-_6$ fluoroalkyle, $COR_d$, CN, $NO_2$, $NR_{9'}R_{10'}$, un hydrogène, un halogène ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes Rb identiques ou différents
- $R_3$ et $R_4$ sont identiques ou différents et représentent un atome d'hydrogène ou un groupe $C_1-_9$ alkyle, $C_3-_9$ cycloalkyle, $C_1-_6$ fluoroalkyle, $-(CH_2)_k-C_{3-9}$ cycloalkyle, $-C_{2-6}$ alkyle-OH, $-(CH_2)_p-C_{1-6}$ alkyloxy, $-(CH_2)_k-C_{3-7}$ cycloalkyle, $-(CH_2)_k-C_{1-6}$ fluoroalkyle, $-(CH_2)_r-O-C_{1-6}$ fluoroalkyle
  Eventuellement les groupes $R_3$ et $R_4$ peuvent former avec l'atome de carbone qui les porte un groupe $C_{3-9}$ cycloalkyle ou un hétérocycle tel que tetrahydrofuranne, tetrahydropyranne, tetrahydrothiopyranne, tetrahydro-1 oxy-thiopyranne ou tetrahydro-1,1 dioxy thiopyranne.
- $R_5$, $R_6$, $R_7$, $R_8$ sont identiques ou différents et représentent soit un atome d'hydrogène soit un groupe $C_{1-6}$ alkyle, $C_{3-7}$ cycloalkyle, $C_{1-6}$ alkyloxy, $-S(O)_g-C_{1-6}$ alkyle, $C_{1-6}$ fluoroalkyle, $C_{1-6}$ fluoroalkyloxy, $-(CH_2)_j-C_{3-9}$ cycloalkyle,

$-(CH_2)_j-C_{3-9}$ cycloalkyle, $-C_{1-6}$ alkyle -OH, $-(CH_2)_j-C_{1-6}$ alkyloxy, $-(CH_2)_j-C_{1-6}$ fluoroalkyle, $-(CH_2)_s-O-C_{1-6}$ fluoroalkyle, $COR_e$, CN, $NR_{11}R_{12}$, ou un halogène ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_c$ identiques ou différents

- $R_a$, $R_d$ et $R_e$ sont identiques ou différents et représentent un groupe $C_{1-6}$ alkyle, $C_{1-6}$ alkyloxy ou $NR_{13}R_{14}$,
- $R_b$ et $R_c$ sont identiques ou différents et représentent un halogène, un groupe $C_{1-6}$ alkyle, $C_{3-7}$ cycloalkyle, $C_{1-6}$ alkyloxy, $-S(O)_u-C_{1-6}$ alkyle, $C_{1-6}$ fluoroalkyle, $C_{1-6}$ fluoroalkyloxy, $-(CH_2)_i-C_{3-7}$ cycloalkyle, OH, $-(CH_2)_i-C_{3-7}$ cycloalkyle, $C_{1-6}$ alkyle-OH, $-(CH_2)_i-C_{1-6}$ alkyloxy, $-(CH_2)_i-C_{1-6}$ fluoroalkyle, $-(CH_2)_t-O-C_{1-6}$ fluoroalkyle, $COR_a$, CN, ou $NR_{15}R_{16}$
- $R_9$, $R_{9'}$, $R_{10}$, $R_{10'}$, $R_{11}$, $R_{12}$, $R_{13}$, $R_{14}$, $R_{15}$ et $R_{16}$ sont identiques ou différents et représentent un atome d'hydrogène, un groupe $C_{1-6}$ alkyle, $C_{3-7}$ cycloalkyle, $-(CH_2)_h-C_{3-7}$cycloalkyle ou $-(CH_2)_h-C_{1-6}$ fluoroalkyle.
  Eventuellement les groupes $R_9$ et $R_{10}$ peuvent former avec l'atome d'azote qui les porte un hétérocycle tel que : azetidine, pyrolidine, pipéridine, azepane, morpholine ou pipérazine. Eventuellement les groupes $R_{9'}$ et $R_{10'}$ peuvent former avec l'atome d'azote qui les porte un hétérocycle tel que : azetidine, pyrolidine, pipéridine, azepane, morpholine ou pipérazine. Eventuellement les groupes $R_{11}$ et $R_{12}$ peuvent former avec l'atome d'azote qui les porte un hétérocycle tel que : azetidine, pyrolidine, pipéridine, azepane, morpholine ou pipérazine. Eventuellement les groupes $R_{13}$ et $R_{14}$ peuvent former avec l'atome d'azote qui les porte un hétérocycle tel que : azetidine, pyrolidine, pipéridine, azepane, morpholine ou pipérazine. Eventuellement les groupes $R_{15}$ et $R_{16}$ peuvent former avec l'atome d'azote qui les porte un hétérocycle tel que : azetidine, pyrolidine, pipéridine, azepane, morpholine ou pipérazine.
- h, i, j, k, l et n sont différents ou identiques et sont égales à 1, 2 ou 3
- f, g, m et u sont différents ou identiques et sont égales à 0, 1 ou 2
- p, q, r, s et t sont différents ou identiques et sont égales à 2, 3 ou 4

ainsi que leurs sels pharmaceutiquement acceptables, solvates ou hydrates et leurs conformères ou rotamères.

Les composés de formule (I) peuvent comporter un ou plusieurs atomes de carbone asymétriques. Ils peuvent donc exister sous forme de mélange d'énantiomères ou de diastéréoisomères. Ces énantiomères, diastéréoisomères ainsi que leurs mélanges y compris les mélanges racémiques font partie de la description.

Les composés de formule (I) peuvent exister à l'état de base ou de sels d'addition à des acides. Ces sels sont avantageusement préparés avec des acides pharmaceutiquement acceptables mais les sels d'autres acides utiles, par exemple pour la purification ou l'isolement des composés de formule (I) font également partie de l'invention. Ces acides peuvent être par exemple l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate. Pour une revue des sels physiologiquement acceptables voir Handbook of Pharmaceutical Salts : Properties, Selection and Use de Stahl et Wermuth (Wiley-VCH, 2002).

Les solvates ou hydrates pourront être obtenus directement à l'issue du procédé de synthèse, le composé (I) étant isolé sous la forme d'un hydrate, par exemple un mono ou hémi-hydrate ou d'un solvate du solvant de réaction ou de purification.

Dans le cadre de la description on entend par :

- $C_{b-c}$ où b et c peuvent prendre des valeurs de 1 à 9, une chaine carbonnée de b à c atomes de carbone, par exemple $C_{1-6}$ une chaine carbonnée pouvant avoir 1 à 6 atomes de carbone
- alkyle, un groupe aliphatique saturé linéaire ou ramifié, par exemple un groupe $C_{1-6}$ alkyle représente une chaine carbonnée de 1 à 6 atomes de carbone, linéaire ou ramifiée, par exemple un méthyle, éthyle, propyle, isopropyle, butyle, isobutyle, terbutyle, pentyle, hexyle
- cycloalkyle, une chaine carbonnée saturée cyclique éventuellement ramifiée, comportant de 3 à 7 atomes de carbone. A titre d'exemple un groupe $C_{3-7}$ cycloalkyle représente une chaine carbonnée de 3 à 7 atomes de carbone par exemple un cyclopropyle, cyclobutyle, cyclopentyle, cyclohexyle, cycloheptyle
- hétérocycle, une chaine hydrocarbonée cyclique ou bicylique, saturée ou insaturée, comprenant un ou plusieurs hétéroatomes choisis parmi O, S et N,
- heteroaryle, un hétérocycle aromatique, par exemple un groupe pyridinyle, pyrazinyle, pyrimidinyle, pyridazinyle, triazinyle, pyrazolyle, isooxazolyle, oxadiazolyle, thiazolyle, thiadiazolyle, triazolyle, imidazolyle
- halogène, un atome de fluore, chlore ou brome
- alkyloxy, un groupe -O-alkyle
- alkylthio, un groupe -S-alkyle
- fluoroalkyle, un groupe alkyle dont un ou plusieurs atomes d'hydrogène ont été remplacés par un atome de fluor
- fluoroalkyloxy, un groupe alkyloxy dont un ou plusieurs atomes d'hydrogène ont été remplacés par un atome de fluor

Est préféré le groupe (A) des composés de formule (I) ci-dessus définis, dans lesquels :

- $R_1$ représente un halogène, un groupe méthyle, éthyle, isopropyle, trifluorométhyle, nitrile, nitro, methoxy, ethoxy, isopropoxy, thiométhyle, thioéthyle, thio isopropyle
- $R_2$ représente un atome d'hydrogène, un halogène, un groupe méthyle, éthyle, isopropyle, trifluorométhyle, nitrile, nitro, methoxy, ethoxy, isopropoxy, thiométhyle, thioéthyle, ou thio isopropyle

et plus particulièrement lorsque que :

- $R_1$ représente un halogène, un groupe méthyle, éthyle, methoxy, ethoxy, thiométhyle, thioéthyle ou trifluorométhyle
- $R_2$ représente un atome d'hydrogène, un halogène, un groupe methoxy, ethoxy, thiométhyle, thioéthyle ou trifluorométhyle

L'invention a pour objet les composés ci-dessous, ainsi que leurs sels pharmaceutiquement acceptables, solvates et hydrates et leurs conformères ou rotamères sont particulièrement préférés :

2-[(2-Bromo-6-methoxy-pyridin-4-ylamino)-methyl]-phenol
2-[(2-Chloro-pyridin-4-ylamino)-methyl]-phenol
2-[(2-Bromo-pyridin-4-ylamino)-methyl]-phenol
2-[(2-Bromo-pyridin-4-ylamino)-methyl]-4-fluoro-phenol
2-[(2-Methoxy-pyridin-4-ylamino)-methyl]-phenol
2-[(2-Trifluoromethyl-pyridin-4-ylamino)-methyl]-phenol
4-Fluoro-2-[(2-methoxy-pyridin-4-ylamino)-methyl]-phenol
2-[(2-Bromo-6-methoxy-pyridin-4-ylamino)-methyl]-4-fluoro-phenol
2-[(2-Bromo-6-methoxy-pyridin-4-ylamino)-methyl]-6-fluoro-phenol
2-[(2-Bromo-6-methoxy-pyridin-4-ylamino)-methyl]-6-methyl-phenol
2-[(2-Bromo-6-methoxy-pyridin-4-ylamino)-methyl]-5-fluoro-phenol
2-[(2-Bromo-6-methoxy-pyridin-4-ylamino)-methyl]-3-fluoro-phenol
2-[(2-Bromo-6-methoxy-pyridin-4-ylamino)-methyl]-3-fluoro-phenol
2-[(2-Chloro-6-methoxy-pyridin-4-ylamino)-methyl]-phenol
2-[(2-Bromo-6-methoxy-pyridin-4-ylamino)-methyl]-5-methyl-phenol
2-[(2-Chloro-6-methoxy-pyridin-4-ylamino)-methyl]-4-fluoro-phenol
2-[(2-Bromo-6-methyl-pyridin-4-ylamino)-methyl]-phenol

Les composés ci-dessous sont aussi décrits

30 2-[(2-Bromo-6-methyl-pyridin-4-ylamino)-methyl]-4-fluoro-phenol
2-[1-(2-Bromo-6-methoxy-pyridin-4-ylamino)-ethyl]-phenol
2-[1-(2-Bromo-6-methoxy-pyridin-4-ylamino)-propyl]-phenol
2-[(2-Bromo-6-ethoxy-pyridin-4-ylamino)-methyl]-phenol
2-[1-(2-Bromo-6-methoxy-pyridin-4-ylamino)-1-methyl-ethyl]-phenol
2-[(2-Bromo-6-methyl-pyridin-4-ylamino)-methyl]-5-fluoro-phenol

La description a également pour objet un procédé de préparation des composés de formule générale (I).
Conformément à l'invention on peut préparer les composés de formule (I) par une des trois méthodes décrites dans le **Schéma 1** ci après.

## Schéma 1

**Méthode 1a**

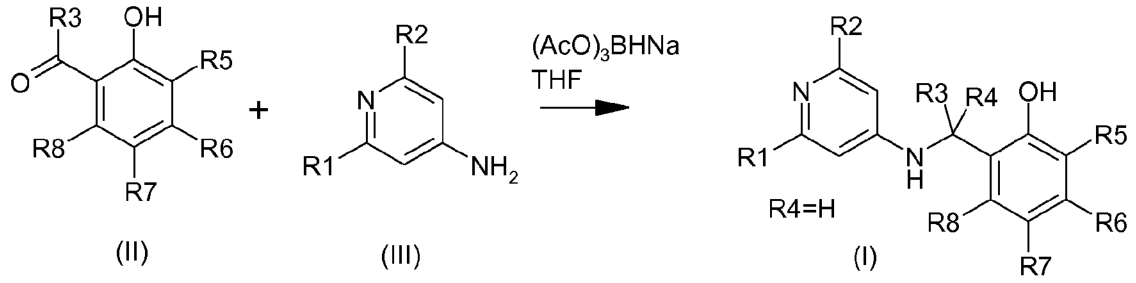


(II) (III) (I)

**Méthode 1b**

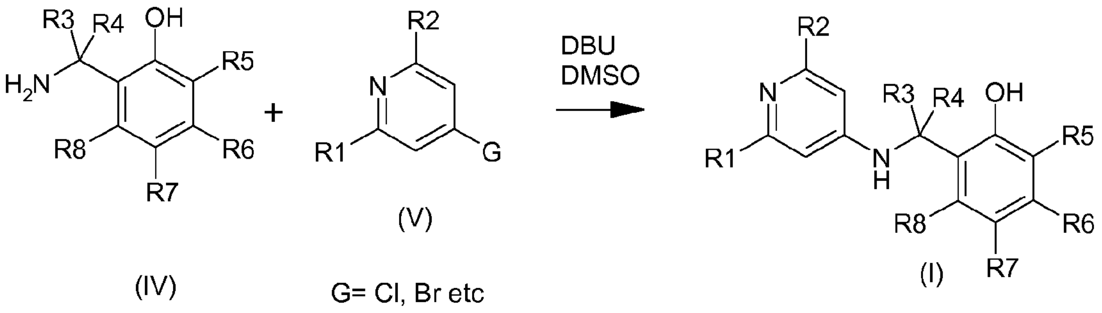


(IV) (V) (I)

G= Cl, Br etc

Les composés phénoliques de formule (I) dans laquelle $R_1$, $R_2$, $R_3$, $R_4$, $R_5$, $R_6$, $R_7$ et $R_8$ sont tels que définis ci-dessus, peuvent être préparés par réaction d'amination réductrice entre un aldéhyde ou une cétone benzylique (II) et une amine (III) en présence d'un agent réducteur, tel que par exemple et de façon non limitante, le triacétoxyborohydrure de sodium, selon la **Méthode 1a** illustrée dans le **Schéma 1** et par analogie, par exemple, aux réactions décrites dans Org. Pro. R. & D. (2006) 971 - 1031.

Les composés phénoliques de formule (I) peuvent être préparés par réaction entre des hétérocycles (V) comportant un groupe partant et des amines benzyliques (IV) en présence d'une base telle que, de façon non limitante, 1, 8- diazabicyclo [5.4.0] undec-7-ene, par exemple dans un solvent tel que le dimethylsulfoxyde tel que décrit par la **Méthode 1b** du **Schéma 1**. Par groupe partant on désigne un groupe bien connu de l'homme de l'art tel que, de façon non limitante, un halogène, un mésylate, un tosylate ou un triflate.

Les groupes fonctionnels éventuellement présents dans les intermédiaires réactionnels utilisés dans le procédé peuvent être protégés, soit de façon permanente, soit de façon temporaire, par des groupes protecteurs qui assurent une synthèse univoque des composés attendus. Les réactions de protection et déprotection sont effectuées selon des techniques bien connues de l'homme de l'art. Par groupe protecteur temporaire des amines, des alcools ou des acides carboxyliques on entend les groupes protecteurs tels que ceux décrits dans « Protective Groups in Organic Chemistry », ed McOmie J. W. F., Plenum Press, 1973, dans « Protective Groups in Organic Synthesis », 2nde édition, Greene T.W. et Wuts P.G.M., ed John Wiley et Sons, 1991 et dans « Protecting Groups », Kocienski P.J., 1994, Georg Thieme Verlag.

Les produits objets de la présente invention sont doués de propriétés pharmacologiques intéressantes; on a constaté notamment qu'ils modulaient l'activité du récepteur aux androgènes.

Des tests donnés dans la partie expérimentale illustrent cette activité modulatrice du récepteur aux androgènes. Les produits objets de la présente invention présentent des activités d'antagonisme ou d'agonisme partiel ou total. Du fait de cette activité, les produits de l'invention peuvent être utilisés comme médicaments chez l'homme ou l'animal.

Ces propriétés rendent les produits de la présente invention utilisables comme médicaments pour le traitement des cancers hormonaux dépendant tels que le cancer de la prostate ou le cancer du sein, ainsi que pour lutter contre l'hyperplasie bénigne de la prostate, la puberté précoce, la virilisation, le syndrome des ovaires polykystiques, syndrome de Stein-Leventhal, la perte de libido, l'endométriose. Les composés présentant une activité d'agonisme partiel ou total peuvent en particulier être utilisés pour traiter les afflictions telles que la perte de masse musculaire (sarcopénie), l'atrophie musculaire, l'impuissance et la stérilité masculine, la différentiation masculine anormale (hermaphrodisme), l'hypogonadisme, l'ostéoporose.

Les produits de l'invention trouvent également leur utilisation cosmétique pour l'hygiène corporelle ou capillaire.

Les produits de l'invention trouvent également leur utilisation dans le traitement de l'hirsutisme, de l'acné, de la séborrhée, la peau grasse de l'alopécie andro génique, de l'hyperpilosité ou hirsutisme, et ils peuvent être utilisés

pour la fabrication d'un médicament pour prévenir et/ou traiter l'hirsutisme, l'alopécie androgénique, de l'hyperpilosité, la dermatite atopique, les désordres de la glande sébacée tels que l'hyperséborrhée, l'acné, la peau grasse ou la dermite séborrhéique.. Les produits de formule (I) peuvent donc être utilisés en dermatologie: ils peuvent être utilisés seuls ou en association. Ils peuvent être associés notamment avec un produit antibiotique tels que les dérivés de l'acide azélaique, fusidique, l'érythromycine ou avec un dérivé des rétinoïdes tel que la tretinoïne pour le traitement de l'acné, ou avec un inhibiteur de la 5 a- réductase tel que le (5alpha,17beta)-N-1,1- diméthyléthyl 3-oxo 4-aza-androst-1-ène 17-carboxamide (ou Finastéride Merck, l3ème édition) ou l'acide azélaïque ou un agent bloquant des récepteurs androgènes pour le traitement de l'acné, de l'alopécie ou de l'hirsutisme, ou avec un produit stimulant la croissance des cheveux tel que le Minoxidil pour le traitement de l'alopécie.

La présente invention a également pour objet à titre de médicament les composés tels que décrits ci-dessus, ainsi que leurs sels pharmaceutiquement acceptables, solvates pharmaceutiquement acceptables et/ou hydrates.

A titre d'illustration et sans aucun caractère limitatif, plusieurs exemples de préparation de composés actifs de formule (I) selon l'invention, sont donnés ci-après, ainsi que des résultats d'activité biologiques de tels composés.

MODES OPERATOIRES

**Exemple 1** : **2-[(2-Bromo-6-methoxy-pyridin-4-ylamino)-methyl]-phenol**

Synthèse selon le Schéma 1, Méthode 1a.

400mg (1.97mmoles) de 2-bromo-6-methoxy-pyridin-4-ylamine (**produits de départs 1)** sont ajoutés à un mélange de 363mg (2.96mmoles, 1.5eq) de 2-hydroxy benzaldéhyde (**produits de départs 2**) et de 184mg (2.96mmoles, 1.5eq) d'acide acétique dans 10mL de THF en présence de tamis moléculaire. Après 5 minutes à température ambiante 835mg (3.94mmoles, 2eq) de triacetoxyborohydrure de sodium sont ajoutés et on laisse agiter 2H à température ambiante. Le milieu réactionnel est dilué avec 50mL d'acétate d'éthyle puis on lave avec 50mL d'une solution de chlorure d'ammonium saturée suivi de trois fois 50mL d'eau. La phase organique est concentrée à sec et le résidu est purifié par chromatographie sur silice en éluant avec un mélange d' heptane/acétate d'éthyle (7/3). 2-[(2-Bromo-6-methoxy-pyridin-4-ylamino)-methyl]-phenol est obtenu sous forme d'un solide beige. Point de fusion = 137°C
RMN 1H (DMSO) 3.69 (s, 3H); 4.17 (d, 2H, J=5.2Hz); 5.81 (s, 1H); 6.45 (s, 1H) 6.75 (t, 1H, J=7.4Hz); 6.82 (d, 1H, J=8Hz); 7.07 (t, 1H, J=7.7Hz); 7.12 (d, 1H, J=7.4Hz); 7.17-7.19 (m, 1 H); 9.64 (s, 1 H).

**Exemple 2 à 14**

Les exemples 2 à 14 sont décrits dans le tableau 1 ci-dessous. Les composés sont synthétisés suivant le mode opératoire ci dessus, en remplaçant les **produits de départs 1 et 2** évoqués dans l'exemple 1 par les produits mentionnés dans le tableau 1.

**Tableau 1**

| Exemple # | Nom IUPAC | Produit de départ 1 | Produit de départ 2 | Point de fusion (°C) | RMN1H-400Mhz (s= singulet, d= doublet, t=triplet, m=multiplet, q=quadruplet, J=constante de couplage en Hz) |
|---|---|---|---|---|---|
| 2 | 2-[(2-Chloro-pyridin-4-ylamino)-methyl]-phenol | 4 Amino 2 chloropyridine | 2-Hydroxy-benzaldehyde | 200 | (DMSO) 4,21 (d, 2H, *J*=5,7Hz); 6,51-6,52 (m, 2H); 6,75 (t, 1H, *J*=7,4Hz); 6,84 (d, 1H, *J*=8,0Hz 7,01-7,14 (m, 2H); 7,28-7,30 (m, 1H); 7,78 (d, 1H, *J*=5,8Hz); 9,65 (s, 1H) |
| 3 | 2-[(2-Bromo-pyridin-4-ylamino)-methyl]-phenol | 2-Bromo-pyridin-4-ylamine | 2-Hydroxy-benzaldehyde | 192 | (DMSO) 4.20 (d, 2H, J= 5.8 Hz); 6.54(d, 1H, J= 4.3 Hz); 6.65 (s, 1H); 6.74 (t, 1H, J= 7.4Hz); 6.84 (d, 1H, J= 8.9 Hz); 7.06-7.13 (m, 2H); 7.26- 7.29 (m, 1H); 7.75 (d, 1H, J= 5.8 Hz); 9.65 (s, 1H). |
| 4 | 2-[(2-Bromo-pyridin-4-ylamino)-methyl]-4-fluoro-phenol | 2-Bromo-pyridin-4-ylamine | 5-Fluoro-2-hydroxy-benzaldehyde | 153 | (DMSO) 4.36 (d, 2H, J= 4.8 Hz); 6.73 (t, 1H, J = 7.4 Hz); 6.81- 6.84 (m, 2H); 7.04-7.08 (m, 2H); 7.14 (d, 1H, J= 7.4 Hz); 7.46-7.54 (m, 2H); 9.60 (s, 1H) |
| 5 | 2-[(2-Methoxypyridin-4-ylamino)-methyl]-phenol | 2-Methoxy-pyridin-4-ylamine | 2-Hydroxy-benzaldehyde | Non déterminé | (DMSO) 3.70 (s, 3H); 4.17 (d, 2H, J= 5.8 Hz); 5.75 (s, 1H); 6.25 (d, 1H, J= 7.8 Hz); 6.73 (t, 1H, J= 7.5Hz); 6.80- 6.87 (m, 2H); 7.05(t, 1H, J= 7.8Hz); 7.11 (d, 1H, J= 8.8 Hz); 7.61 (d, 1H, J= 5.8 Hz), 9.59 (s, 1H). |
| 6 | 2-[(2-Bromo-6-methoxy-pyridin-4-ylamino)-methyl]-4-fluoro-phenol | *2-Bromo-6-methoxy-pyridin-4-ylamine* | *5-Fluoro-2-hydroxy-benzaldehyde* | 127 | (DMSO) 3.70 (s, 3H); 4.17-4.18 (d, 2H, J= 4 Hz); 5.80 (s, 1H); 6.46 (s, 1H); 6.79-6.83 (m, 1H); 6.88- 6.93 (m, 2H); 7.21-7.24 (m, 1H); 9.71 (s, 1H). |
| 7 | 2-[(2-Bromo-6-methyl-pyridin-4-ylamino)-methyl]-phenol | *salicylaldéhyde* | *2-bromo-6-methyl-pyridin-4-amine* | 205 | (DMSO) 2.2 (s, 3H); 4.18-4.2 (d, 2H, J=8 Hz); 6,4-6,41 (d, 1H, J=4Hz); 6,49 (s, 1H); 6,73-6,77 (q, 1H, 16Hz); 6,82-6,84 (d, 1H, J=8Hz); 7,06-7,16 (m, 3H); 9.64 (s, 1H) |
| 8 | 2-[(2-Bromo-6-methyl-pyridin-4-ylamino)-methyl]-4-fluoro-phenol | *5-Fluoro-2-hydroxy-benzaldéhyde* | *2-bromo-6-methyl-pyridin-4-amine* | 198 | (DMSO) 2.24 (s, 3H); 4.22-4.23 (d, 2H, J=4 Hz); 6,44 (s, 1H); 6,52 (s, 1H); 6,83-6,86 (q, 13H, J=12Hz); 6,91-6,96 (m, 2H); 7,06-7,16 (m, 3H); 9.64 (s, 1H); 7.20-7.22 (d, 1H, J=8Hz), 9.8 (s, 1H) |

(suite)

| Exemple # | Nom IUPAC | Produit de départ 1 | Produit de départ 2 | Point de fusion (°C) | RMN1H-400Mhz (s= singulet, d= doublet, t=triplet, m=multiplet, q=quadruplet, J=constante de couplage en Hz) |
|---|---|---|---|---|---|
| 9 | 2-[(2-Bromo-6-methyl-pyridin-4-ylamino)-methyl]-5-fluoro-phenol | *4-Fluoro-2-hydroxy-benzaldéhyde* | *2-bromo-6-methyl-pyridin-4-amine* | 200 | (DMSO) 2.2 (s, 3H); 4.15-4.16 (d, 2H, J=4 Hz); 6,40 (s, 1H); 6,49 (s, 1H); 6,57-6,64 (m, 2H); 7,1-7,16 (m, 2H); 9.64 (s, 1H); 7.20-7.22 (d, 1H, J=8Hz), 10.2 (s, 1H) |
| 10 | 2-[(2-Bromo-6-methoxy-pyridin-4-ylamino)-methyl]-6-fluoro-phenol | *2 bromo-6-methoxypyridine-4-amine* | *2 hydroxy 3 fluorobenzaldehyde* | 143 | (DMSO) 3.70 (s, 3H); 4.23-4.25 (d, 2H, J =8Hz); 5.80 (s, 1H); 6.46 (s, 1H); 6.75-6.80 (m, 1H); 6.95-6.97(d, 1H, J= 8Hz); 7.04-7.09 (tr, 1H); 7.22-7.24 (tr, 1H); 9.77 (s, 1H) |
| 11 | 2-[(2-Bromo-6-methoxy-pyridin-4-ylamino)-methyl]-5-fluoro-phenol | *2 bromo-6-methoxypyridine-4-amine* | *2 hydroxy 4 fluorobenzaldehyde* | 192 | (DMSO) 3.70 (s, 3H); 4.13-4.14 (d, 2H, J =4Hz); 5.81 (s, 1H); 6.45 (s, 1H); 6.57-6.63 (m, 2H); 7.12-7.17 (m, 2H); 10.12 (s, 1H) |
| 12 | 2-[(2-Bromo-6-ethoxy-pyridin-4-ylamino)-methyl]-phenol | *2 bromo-6-ethoxypyridine-4-amine* | *Salycaldehyde* | 156 | (DMSO) 1.20-1.24 (tr, 3H); 4.09-4.14 (q, 2H, J=8Hz, J'=12Hz); 4.16-4.18 (d, 2H, J =8Hz); 5.77 (s, 1H); 6.44 (s, 1H); 6.74-6.77 (tr, 1H); 6.82-6.84(d, 1H, J= 8Hz); 7.05-7.18 (m, 3H); 9.63 (s, 1H) |
| 13 | 2-[(2-Bromo-6-methoxy-pyridin-4-ylamino)-methyl]-5-methyl-phenol | *2 bromo-6-methoxypyridine-4-amine* | *2 hydroxy 4 methylbenzaldehyde* | 164 | (CDCl3) 2.31 (s, 3H); 3.88 (s, 1H); 4.30 (s, 2H); 4.50 (s, 1H); 5.91 (s, 1H); 6.44 (s, 1H); 6.66 (s, 1H); 6.73-6.75 (d, 1H, J=7.7Hz); 7.08-7.10 (d, 1H, J=7.6Hz) |
| 14 | 2-[(2-Bromo-6-methoxy-pyridin-4-ylamino)-methyl]-6-methyl-phenol | *2 bromo-6-methoxypyridine-4-amine* | *2 hydroxy 3 methylbenzaldehyde* | 149 | (DMSO) 2.19 (s, 3H); 3.70 (s, 3H); 4.22-4.23 (d, 2H, J =5.4Hz); 5.80 (s, 1H); 6.45 (s, 1H); 6.70-6.73 (tr, 1H); 6.96-6.99 (m, 2H); 7.13-7.15 (m, 1H); 8.50 (s, 1H) |

**Exemple 15** : **2-[(2-Trifluoromethyl-pyridin-4-ylamino)-methyl]-phenol**

Synthèse selon le Schéma 1, Méthode 1b.

Dans un tube micro onde, on introduit 300mg (1.65mmoles) de 4 chloro 2(trifluoromethyl) pyridine, auquel on ajoute 5ml de diméthylsulfoxyde, 251 mg (1.65mmoles, 1eq) de 1, 8-diazabicyclo [5.4.0] undec-7-ene, et 406mg (3.3mmoles, 2eq) de 2-aminomethyl-phenol, on chauffe au micro onde à 150°C pendant 30 minutes. Le milieu réactionnel est dilué avec 50ml d'acétate d'éthyle, on lave avec 50ml d'une solution de chlorure d'ammonium saturée, puis avec trois fois 50ml d'eau. La phase organique est concentrée à sec et le résidu est purifié par chromatographie sur silice en éluant avec un mélange heptane/acétate d'éthyle (7/3). On obtient 2-[(2-Trifluoromethyl-pyridin-4-ylamino)-methyl]-phenol sous la forme d'un solide blanc.
RMN 1 H (DMSO) 4.27 (d, 2H, J=5.5Hz); 6.69 (s, 1 H); 6.76 (t, 1 H, J=7.2Hz); 6.84 (d, 1 H, J=7.9Hz); 6.97 (s, 1H); 7.09 (t, 1 H, J=6.8Hz); 7.15 (d, 1 H, J=7.3Hz); 7.47 (s, 1 H); 8.12 (d, 1 H, J=5.6Hz); 9.68 (s, 1 H).

Tous les spectres de RMN (résonance magnétique nucléaire) sont en accord avec les structures proposées. Les déplacements chimiques sont exprimés en partie par million. La référence interne est le tetraméthylsilane. Les abréviations suivantes sont utilisées : CDCl3 = chloroforme deutérié, DMSO = diméthylsulphoxide deutérié, CD3OD = méthanol deutérié.

**Exemple 16** : **Tests biologiques**

Les composés selon l'invention présentent des propriétés inhibitrices des récepteurs de type AR. Cette activité inhibitrice des récepteurs AR est mesurée dans un test de transactivation par les constantes de dissociation KdR (repos), KdA (actif) et Kdapp (apparent) d'après la méthode exposée dans J. Molecular Biology (1965), 12(1), 88-118, Monod J. *et al.*

Par inhibiteur des récepteurs de type ARs, on entend selon l'invention tout composé qui présente une constante de dissociation Kdapp inférieure ou égale à 1 μM, et un rapport KdR / KdA ≤ 10, dans un test de transactivation.
Les composés préférés de la présente invention présentent une constante de dissociation inférieure ou égale à 500 nM, et avantageusement inférieur ou égal à 100 nM.

Le test de transactivation est réalisé dans la lignée cellulaire PALM (PC3 Androgene receptor Luciferase MMTV) qui est un transfectant stable contenant les plasmides PMMTV-neo-Luc (gène reporter) et pSG5puro-AR.
Dans cette étude, l'affinité de chaque produit pour les 2 états des récepteurs (KdR et KdA) est déterminée ainsi qu'un Kd apparent (KdApp). Cette constante est une résultante des 2 Kd mais dépend également de l'équilibre initial du récepteur entre l'état actif et l'état repos ($L_0$) et de son taux d'expression. Sa détermination se fait par la formule suivante:

$$1/KdApp=(L0/(1+L0))x(1/KdR) +(1/(1+L0))x(1/KdA)$$

Pour déterminer ces constantes, des « courbes croisées » du produit à tester contre un agoniste de référence, le methyltrienolone, sont réalisées en plaque 96 puits. Le produit à tester est utilisé à 10 concentrations et l'agoniste de référence à 7 concentrations.

A titre illustratif, un Kdapp de 20 nM est obtenu pour le composé (1), un Kdapp de 4nM est obtenu pour le composé (2), un Kdapp de 20 nM est obtenu pour le composé (4), un Kdapp de 50 nM est obtenu pour le composé (5).

## Revendications

**1.** Composés choisis parmi les composés ci-dessous, leurs sels pharmaceutiquement acceptables, solvates, hydrates, conformères et rotamères :

2-[(2-Bromo-6-methoxypyridin-4-ylamino)methyl]phenol,
2-[(2-Chloropyridin-4-ylamino)methyl]phenol,
2-[(2-Bromopyridin-4-ylamino)methyl]phenol,
2-[(2-Bromopyridin-4-ylamino)methyl]-4-fluorophenol,
2-[(2-Methoxypyridin-4-ylamino)methyl]phenol,
2-[(2-Trifluoromethylpyridin-4-ylamino)methyl]phenol,
4-Fluoro-2-[(2-methoxypyridin-4-ylamino)methyl]phenol,
2-[(2-Bromo-6-methoxypyridin-4-ylamino)methyl]-4-fluorophenol,

2-[(2-Bromo-6-methoxypyridin-4-ylamino)methyl]-6-fluorophenol,
2-[(2-Bromo-6-methoxypyridin-4-ylamino)methyl]-6-methylphenol,
2-[(2-Bromo-6-methoxypyridin-4-ylamino)methyl]-5-fluorophenol,
2-[(2-Bromo-6-methoxypyridin-4-ylamino)methyl]-3-fluorophenol,
2-[(2-Chloro-6-methoxypyridin-4-ylamino)methyl]phenol,
2-[(2-Bromo-6-methoxypyridin-4-ylamino)methyl]-5-methylphenol,
2-[(2-Chloro-6-methoxypyridin-4-ylamino)methyl]-4-fluorophenol, et
2-[(2-Bromo-6-ethoxypyridin-4-ylamino)methyl]phenol.

**2.** Composés selon la revendication 1, **caractérisés en ce qu'**ils sont choisis parmi :

2-[(2-Bromo-6-methoxypyridin-4-ylamino)methyl]phenol,
2-[(2-Chloropyridin-4-ylamino)methyl]phenol,
2-[(2-Bromopyridin-4-ylamino)methyl]-4-fluorophenol, et
2-[(2-Methoxypyridin-4-ylamino)methyl]phenol.

**3.** Composés selon la revendication 1 ou 2, en tant que médicaments.

**4.** Utilisation cosmétique d'un composé tel que défini selon la revendication 1 ou 2 pour l'hygiène corporelle ou capillaire.

**5.** Utilisation d'un composé selon la revendication 1 ou 2 pour la fabrication d'un médicament pour prévenir et/ou traiter l'hirsutisme, l'apolécie androgénique, l'hyperpilosité, la dermatite atopique, les désordres de la glande sébacée, l'acné, la peau grasse ou la dermite séborrhéique.

**6.** Utilisation selon la revendication 5, dans laquelle les désordres de la glande sébacée sont l'hyperséborrhée.

**7.** Utilisation d'un composé selon la revendication 1 ou 2 pour la fabrication d'un médicament pour traiter l'acné.

**Patentansprüche**

**1.** Verbindungen, ausgewählt aus den nachstehenden Verbindungen, ihre pharmazeutisch verträglichen Salze, Solvate, Hydrate, Konformere und Rotamere:

2-[(2-Brom-6-methoxypyridin-4-ylamino)methyl]phenol,
2-[(2-Chlorpyridin-4-ylamino)methyl]phenol,
2-[(2-Brompyridin-4-ylamino)methyl]phenol,
2-[(2-Brompyridin-4-ylamino)methyl]-4-fluorphenol,
2-[(2-Methoxypyridin-4-ylamino)methyl]phenol,
2-[(2-Trifluormethylpyridin-4-ylamino)methyl]phenol,
4-Fluor-2-[(2-methoxypyridin-4-ylamino)methyl]phenol,
2-[(2-Brom-6-methoxypyridin-4-ylamino)methyl]-4-fluorphenol,
2-[(2-Brom-6-methoxypyridin-4-ylamino)methyl]-6-fluorphenol,
2-[(2-Brom-6-methoxypyridin-4-ylamino)methyl]-6-methylphenol,
2-[(2-Brom-6-methoxypyridin-4-ylamino)methyl]-5-fluorphenol,
2-[(2-Brom-6-methoxypyridin-4-ylamino)methyl]-3-fluorphenol,
2-[(2-Chlor-6-methoxypyridin-4-ylamino)methyl]phenol,
2-[(2-Brom-6-methoxypyridin-4-ylamino)methyl]-5-methylphenol,
2-[(2-Chlor-6-methoxypyridin-4-ylamino)methyl]-4-fluorphenol, und
2-[(2-Brom-6-ethoxypyridin-4-ylamino)methyl]phenol.

**2.** Verbindungen gemäß Anspruch 1, **dadurch gekennzeichnet, dass** sie ausgewählt sind aus:

2-[(2-Brom-6-methoxypyridin-4-ylamino)methyl]phenol,
2-[(2-Chlorpyridin-4-ylamino)methyl]phenol,
2-[(2-Brompyridin-4-ylamino)methyl]-4-fluorphenol, und
2-[(2-Methoxypyridin-4-ylamino)methyl]phenol.

**3.** Verbindungen gemäß Anspruch 1 oder 2 als Medikamente.

**4.** Kosmetische Verwendung einer gemäß Anspruch 1 oder 2 definierten Verbindung für die Körper- oder Haarhygiene.

**5.** Verwendung einer Verbindung gemäß Anspruch 1 oder 2 für die Herstellung eines Medikaments für die Prävention und/oder Behandlung von Hirsutismus, androgenetischer Apolezie, Hyperpilosität, atopischer Dermatitis, Störungen der Talgdrüse, Akne, fettiger Haut oder seborrhoischer Dermatitis.

**6.** Verwendung gemäß Anspruch 5, wobei die Störungen der Talgdrüse die Hyperseborrhoe sind.

**7.** Verwendung einer Verbindung gemäß Anspruch 1 oder 2 für die Herstellung eines Medikaments für die Behandlung von Akne.

## Claims

**1.** Compounds chosen from the compounds below, pharmaceutically acceptable salts, solvates, hydrates, conformers and rotamers thereof:

2-[(2-Bromo-6-methoxypyridin-4-ylamino)methyl]phenol
2-[(2-Chloropyridin-4-ylamino)methyl]phenol
2-[(2-Bromopyridin-4-ylamino)methyl]phenol
2-[(2-Bromopyridin-4-ylamino)methyl]-4-fluorophenol
2-[(2-Methoxypyridin-4-ylamino)methyl]phenol
2-[(2-Trifluoromethylpyridin-4-ylamino)methyl]phenol
4-Fluoro-2-[(2-methoxypyridin-4-ylamino)methyl]phenol
2-[(2-Bromo-6-methoxypyridin-4-ylamino)methyl]-4-fluorophenol
2-[(2-Bromo-6-methoxypyridin-4-ylamino)methyl]-6-fluorophenol
2-[(2-Bromo-6-methoxypyridin-4-ylamino)methyl]-6-methylphenol
2-[(2-Bromo-6-methoxypyridin-4-ylamino)methyl]-5-fluorophenol
2-[(2-Bromo-6-methoxypyridin-4-ylamino)methyl]-3-fluorophenol
2-[(2-Chloro-6-methoxypyridin-4-ylamino)methyl]phenol
2-[(2-Bromo-6-methoxypyridin-4-ylamino)methyl]-5-methylphenol
2-[(2-Chloro-6-methoxypyridin-4-ylamino)methyl]-4-fluorophenol, and
2-[(2-Bromo-6-ethoxypyridin-4-ylamino)methyl]phenol.

**2.** Compounds according to claim 1, **characterized in that** they are chosen from:

2-[(2-Bromo-6-methoxypyridin-4-ylamino)methyl]phenol
2-[(2-Chloropyridin-4-ylamino)methyl]phenol
2-[(2-Bromopyridin-4-ylamino)methyl]-4-fluorophenol, and
2-[(2-Methoxypyridin-4-ylamino)methyl]phenol

**3.** Compounds according to claim 1 or 2, as medicaments.

**4.** Cosmetic use of a compound as defined according to Claim 1 or 2, for body or hair hygiene.

**5.** Use of a compound according to Claim 1 or 2, for the production of a medicament for preventing and/or treating hirsutism, androgenic alopecia, hyperpilosity, atopic dermatitis, sebaceous gland disorders, acne, oily skin or seborrhoeic dermatitis.

**6.** Use of a compound according to Claim 5, wherein sebaceous gland disorders are hyperseborrhoea.

**7.** Use of a compound according to claim 1 or 2, for the production of a medicament for treating acne.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 580459 A
- WO 200542464 A


### Littérature non-brevet citée dans la description


- Handbook of Pharmaceutical Salts : Properties, Selection and Use. 2002
- *Org. Pro. R. & D.,* 2006, 971-1031
- Protective Groups in Organic Chemistry. Plenum Press, 1973
- **GREENE T.W. ; WUTS P.G.M.** Protective Groups in Organic Synthesis. 1991
- **KOCIENSKI P.J.** Protecting Groups. Georg Thieme Verlag, 1994
- **MONOD J.** *J. Molecular Biology,* 1965, vol. 12 ((1)), 88-118